(19) Europäisches Patentamt
European Patent Office
Office européen des brevets

(11) Numéro de publication: **0 446 758 B1**

(12) **FASCICULE DE BREVET EUROPEEN**

(45) Date de publication de fascicule du brevet: **14.12.94**

(51) Int. Cl.[5]: **C07C 13/47**, C07C 2/86

(21) Numéro de dépôt: **91103275.3**

(22) Date de dépôt: **05.03.91**

# (54) Procédé pour préparer un produit aromatique alkyle avec une zéolithe d'alkylation et une zéolithe de déalkylation.

(30) Priorité: **13.03.90 FR 9003309**

(43) Date de publication de la demande:
**18.09.91 Bulletin 91/38**

(45) Mention de la délivrance du brevet:
**14.12.94 Bulletin 94/50**

(84) Etats contractants désignés:
**AT BE CH DE DK ES FR GB GR IT LI LU NL SE**

(56) Documents cités:
**WO-A-91/01959**
**US-A- 4 230 894**

**ANNALES DE CHEMIE, vol. 10, no. 11, 1929, pages 535-582, Paris, FR; D. DOBROUX:"Condensation du cyclohexène avec le naphtalène et la tétrahydronaphtalène"**

(73) Titulaire: **MICHELIN RECHERCHE ET TECHNIQUE S.A.**
**Euro-résidence**
**Grand-Places 14 A**
**CH-1700 Fribourg (CH)**

(72) Inventeur: **Solofo, Jonis**
**571, avenue Comté de Nice**
**F-34080 Montpellier (FR)**
Inventeur: **Moreau, Patrice**
**30, rue des Oliviers**
**F-34980 Saint-Gely-du-Fesc (FR)**
Inventeur: **Geneste, Patrick**
**25, avenue Chancel**
**F-34000 Montpellier (FR)**
Inventeur: **Finiels, Annie**
**254, rue du Pré-aux-Clercs**
**F-34090 Montpellier (FR)**

(74) Mandataire: **Doussaint, Jean-Marie et al**
**Michelin & Cie**
**Service SRK Brevets**
**23, Place des Carmes**
**F-63040 Clermont-Ferrand Cédex (FR)**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).



Rank Xerox (UK) Business Services
(3.10/3.09/3.3.3)

## Description

L'invention concerne un procédé permettant d'alkyler des composés, notamment des composés aromatiques, en utilisant des zéolithes comme catalyseurs.

La production de composés aromatiques contenant des groupes alkyles cycliques en utilisant des halogénures d'aluminium est connue, par exemple la synthèse de mono ou dicyclohexylnaphtalènes avec $AlCl_3$, de telles réactions étant décrites en particulier dans les publications suivantes :

- D. Bodroux, Annales de Chimie 1929, (10) 11, 535 ;
- Charles C. Price, J. Am. Chem. Soc, 1943, 65 , 439;
- E.S. Pokrovskaya, J. Gen. Chem (URSS), 1939, 9 , 1953 .

Ce procédé conduit à un faible rendement, à une absence de sélectivité, d'autre part il n'est pas économique.

Les brevets ou demandes de brevets DE-C-638 756, DE-A 2 208 363, US-A-2 229 018, et US-A-3 786 106 décrivent l'alkylation de composés aromatiques par des oléfines, par exemple cycliques, en présence de catalyseurs qui sont des argiles, par exemple la montmorillonite, du kaolin, des hydrosilicates, des terres décolorantes. Ces procédés ne sont pas sélectifs.

Les documents US-A-2 904 607, US-A-3 641 177, US-A-4 393 262, DE-A-3 334 084, EP-A-280 055, J. Catal. 1986, 101, page 273 décrivent l'alkylation de produits aromatiques en utilisant des zéolithes comme catalyseurs, avec des agents d'alkylation linéaires, comme par exemple des oléfines ayant de 2 à 4 atomes de carbone, ou le méthanol. Ces documents ne décrivent pas l'alkylation de produits aromatiques avec des groupes alkyles cycliques.

La demande de brevet DE-A-1 934 426 décrit un procédé continu pour préparer des composés aromatiques alkylés. Ce document cite de nombreux catalyseurs de type zéolithique, par exemple des zéolithes naturelles, comme la gmelinite, la dachiardite, la faujasite, l'heulandite, la mordénite, ou des zéolithes synthétiques comme les zéolithes oméga, L et Y. Ce document cite aussi de nombreux composés aromatiques et une grande variété d'agents d'alkylation. Ce procédé est réalisé en deux étapes. Dans une première étape on réalise l'alkylation avec un agent d'alkylation en présence de zéolithe en suspension dans le milieu liquide. L'alkylation n'est pas sélective et on obtient un mélange de mono et de polyalkylaromatiques. Pour remédier à cette absence de sélectivité, dans une deuxième étape le milieu réactionnel provenant de la première étape est mis au contact d'une zéolithe, sans qu'il y ait de suspension, et sans agent alkylant, de façon à effectuer une transalkylation. Malgré cette deuxième étape, la sélectivité globale à la fin de ces deux étapes reste faible.

Le brevet US 4 230 894 décrit l'alkylation non sélective de benzène ou d'alkylbenzène, pour obtenir un mélange d'isomères que l'on met au contact d'une zéolithe de façon à craquer ou à transalkyler sélectivement l'isomère 1,4-dialkylbenzène afin d'enrichir le mélange en isomère 1,3-dialkylbenzène.

La demande de brevet PCT/CH 90/00178 incorporée ici par référence décrit un procédé permettant de cycloalkyler le naphtalène avec au moins un groupe cyclique, ce procédé étant caractérisé par les points suivants :

a) on utilise comme catalyseur au moins une zéolithe de structure faujasite ayant des ouvertures de pores supérieures à 6,7 Å, le rapport en poids silice/alumine de cette zéolithe étant supérieur à 2,5 et son taux résiduel en ion(s) alcalin(s) étant inférieur à 3 % en poids ;

b) la réaction est effectuée en discontinu en phase hétérogène liquide/solide à une température comprise entre 20 ° C et 250 ° C, sous une pression au plus égale à 30 bars, la/ou les zéolithes étant en suspension dans le milieu réactionnel ;

c) le rapport molaire

$$\frac{\text{agent alkylant}}{\text{naphtalène}}$$

est au moins égal à 1.

L'objet de l'invention est de proposer un procédé pour préparer un produit aromatique alkylé par alkylation d'au moins un composé aromatique en utilisant au moins une zéolithe comme catalyseur, cette alkylation donnant plusieurs produits aromatiques alkylés. Ce procédé se caractérise par des rendements élevés en produit aromatique alkylé désiré grâce à la transformation des sous-produits de la réaction.

Le procédé conforme à l'invention se caractérise par les points suivants :

a) on fait réagir le composé aromatique de départ avec au moins un agent alkylant en présence d'au moins une zéolithe comme catalyseur dite "zéolithe d'alkylation" ;

b) on sépare ensuite par cristallisation le produit aromatique alkylé désiré à partir des produits de l'alkylation ;

c) on soumet la partie non cristallisée des produits aromatiques alkylés à une déalkylation en présence d'au moins une zéolithe comme catalyseur, dite "zéolithe de déalkylation", de façon à régénérer le composé aromatique de départ ;

d) on recycle le composé aromatique régénéré pour le faire réagir à nouveau avec l'agent alkylant, en présence de la zéolithe d'alkylation.

Le procédé conforme à l'invention est particulièrement adapté à la cycloalkylation du naphtalène, pour obtenir les dicycloalkyl-2,6 naphtalènes.

Les réactifs d'alkylation doivent au moins comporter une double liaison, ou un groupe réactif, par exemple un halogène, un groupe OH ; de tels réactifs étant par exemple le cyclohexène, le chloro ou le bromocyclohexane, le cyclohexanol.

Les zéolithes utilisées dans le procédé conforme à l'invention peuvent être sous forme protonique ou échangées par des cations, notamment des cations de terres rares (par exemple La, Ce, Pr, Gd, Yb).

Parmi les composés aromatiques alkylés obtenus par le procédé précédemment décrit, le dicyclohexyl-2,6 naphtalène est très important dans la chimie des polymères car il permet d'obtenir par oxydation des composés contenant des groupes hydroxyles ou carboxyliques en position 2,6, ces produits servant notamment à la synthèse de polyesters ou de polyamides aromatiques.

L'invention sera aisément comprise à l'aide des exemples qui suivent, et des figures, relatives à ces exemples.

Sur le dessin :

- la figure 1 représente le schéma d'un procédé conforme à l'invention ;
- les figures 2 et 3 représentent chacune un schéma partiel correspondant à une modification du schéma représenté à la figure 1, selon d'autres variantes du procédé conforme à l'invention.

La figure 1 représente le schéma général d'un procédé conforme à l'invention. Ce procédé consiste essentiellement à effectuer la cyclohexylation du naphtalène par réaction de bromocyclohexane sur le naphtalène en présence d'une zéolithe comme catalyseur, pour obtenir du dicyclohexyl-2,6 naphtalène, puis à effectuer la déalkylation ultérieure des sous-produits alkylés du naphtalène, en présence d'une zéolithe comme catalyseur, pour régénérer le naphtalène que l'on recycle dans l'étape d'alkylation.

La figure 2 représente le schéma d'une purification éventuelle du dicyclohexyl-2,6 naphtalène et la figure 3 représente le schéma d'une purification éventuelle effectuée sur le naphtalène, après déalkylation, avant recyclage pour alkylation. Ces figures 2 et 3 concernent des modifications pouvant être apportées au procédé conforme à la figure 1, et ces modifications seront décrites ultérieurement.

Dans un but de simplification, les produits utilisés ou obtenus, dans les procédés décrits, sont représentés sur les schémas 1 à 3 par des lignes qui comportent des flèches pour symboliser la circulation de ces produits.

On utilise, dans la description qui suit, la même zéolithe pour la réaction d'alkylation et pour la réaction de déalkylation. Cette zéolithe a la dénomination ZF-520, elle est commercialisée par la société Zéocat.

Les propriétés de cette zéolithe de structure Faujasite, utilisée sous forme d'une poudre, sont données dans le tableau 1 suivant :

Tableau 1

| % en poids | | | Rapport molaire $S_iO_2/Al_2O_3$ | Surface spécifique (m2/g) | Ouverture des pores (Å) |
|---|---|---|---|---|---|
| $S_iO_2$ | $Al_2O_3$ | $Na_2O$ | | | |
| 95,8 | 4,1 | 0,14 | 40 | 800 | 8 |

Avant utilisation, cette zéolithe est activée en la chauffant dans un four de la façon suivante :
La montée de la température du four est de 20 °C à 300 °C à raison de 50 °C/heure. On maintient la température à 300 °C pendant 6 heures, puis on refroidit jusqu'à 200 °C à raison de 40 °C/heure, ce traitement thermique de calcination est conduit sous un courant d'air de 200 $cm^3$/minute.

Les analyses des produits sont effectuées avec un chromatographe en phase gazeuse DELSI série 330 équipé d'une colonne capillaire OV1 de 25 m, muni d'un détecteur à ionisation de flamme. Il est couplé à un intégrateur DELSI ENICA 21. Le nitrobenzène est utilisé comme étalon interne, et il est rajouté dans les prélèvements servant à l'analyse.

Les conditions d'analyse chromatographique sont les suivantes :

- température de l'injecteur : 330 °C ;
- température du détecteur : 320 °C ;
- programmation de la température du four : chauffage de 100 °C à 280 °C, la vitesse de chauffage étant de 15 °C/min ;
- pression de l'hydrogène servant de gaz vecteur : 0,65 bar.

La détermination de la structure des produits de la réaction est effectuée par un ensemble constitué par un chromatographe en phase gazeuse couplé avec un spectromètre de masse. Le chromatographe est équipé d'une colonne capillaire de type OV1 de 25 m.

Les conditions d'analyse avec cet ensemble sont les suivantes :

- température de l'injecteur : 250 °C ;
- température du détecteur : 250 °C ;
- programmation de la température du four : chauffage de 100 °C à 280 °C, avec une montée en température de 10 °C/min ;
- pression de l'hélium utilisé comme gaz vecteur : 0,5 bar.

Pour simplifier la description, les abréviations utilisées dans cette description sont les suivantes :
Naphtalène : NP ; Bromocyclohexane : BCH ; Cyclohexane : CHA ; dicyclohexyl-2,6 naphtalène : 2,6 DCN ; monocyclohexylnaphtalènes : MCN ; dicyclohexylnaphtalènes : DCN ; Cyclohexène : CHE ; zéolithe : ZE.

1 - Alkylation du naphtalène et séparation du dicyclohexyl-2,6 naphtalène

Le procédé mis en oeuvre lors de cette alkylation est conforme à la demande de brevet PCT/CH 90/00178 précitée.

L'alkylation est réalisée dans le réacteur statique $R_1$ qui est un autoclave de 100 ml (100 $cm^3$) de la société BURTON-CORBLIN comportant un agitateur rotatif interne à entraînement magnétique, un manomètre et un appareil pour mesurer la vitesse de rotation.

L'autoclave $R_1$ est chauffé par un four dont la régulation de température est assurée par un régulateur de la société SOTELEM.

On introduit dans le réacteur $R_1$ du napthalène (NP), du bromocyclohexane (BCH), du cyclohexane (CHA), de la zéolithe ZF-520 (ZE), et un mélange de récupération contenant divers composés naphtaléniques autres que NP, ceci de la façon suivante :

- NP introduit 1 : 6,4 g (50 mmoles), soit :
  - . NP d'appoint $1_a$ : 3,3 g (25,8 mmoles) ;
  - . NP de récupération $1_b$ : 3,1 g (24,2 mmoles) ;
- BCH introduit 2 : 16,2 g (100 mmoles), soit ;
  - . BCH d'appoint $2_a$ : 14,0 g (86,4 mmoles) ;
  - . BCH de récupération $2_b$ : 2,2 g (13,6 mmoles) ;
- CHA introduit 3 : 50 $cm^3$ provenant entièrement de la récupération ;
- ZE introduite 4 : 1,0 g provenant entièrement de la récupération ;
- Mélange de récupération $1_c$ (3,5 g) contenant divers composés naphtaléniques autres que NP. Ce mélange constitue avec $1_b$ un produit brut de déalkylation récupéré.

Cette introduction est effectuée dans les conditions ambiantes, la température et la pression dans le réacteur étant alors respectivement d'environ 20 °C et 1 bar.

Les récupérations précédemment mentionnées ainsi que les récupérations qui suivent, correspondent à une préparation précédente conforme à l'invention, étant donné que le procédé décrit est discontinu.

On agite alors le mélange réactionnel dans le réacteur $R_1$ avec une vitesse de 680 tours/minute, après fermeture du réacteur. On obtient ainsi une suspension du catalyseur dans le milieu réactionnel. On porte la température du réacteur de 20 °C environ à 200 °C en 10 minutes, et on arrête l'agitation et le chauffage dès que la température du réacteur atteint 200 °C, la pression autogène dans le réacteur étant alors de 15 bars. Les conditions maximales de température et de pression dans le réacteur, lors de l'alkylation, étant

donc respectivement 200 ° C et 15 bars.

La réaction d'alkylation réalisée en phase hétérogène liquide/solide dans le réacteur $R_1$ est la suivante :

NP + BCH → 2,6 DCN + MCN + DCN autres que 2,6 DCN + HBr

CHA est un solvant de NP, de BCH et des produits aromatiques alkylés.

On laisse alors le réacteur $R_1$ se refroidir. Lorsque sa température atteint 20 ° C, on l'ouvre et HBr gazeux 5 produit par l'alkylation s'échappe à l'extérieur du réacteur $R_1$.

Le mélange réactionnel 6 est alors introduit dans le filtre $F_1$. Ce filtre $F_1$ à deux étages est constitué de deux filtres élémentaires.

Le premier filtre $F_{1a}$ dans la partie supérieure de $F_1$, est constitué de verre fritté, le deuxième filtre $F_{1b}$ dans la partie inférieure, est constitué par du verre fritté, supportant du charbon actif.

Le filtre $F_{1a}$ permet de séparer la ZE que l'on lave avec 50 $cm^3$ de CHA de récupération 7. Après lavage la ZE 8 est introduite dans le four $R_2$. Le four $R_2$ permet de régénérer la ZE en lui faisant subir le traitement d'activation précédemment décrit. La ZE 4 ainsi régénérée est ensuite introduite dans le réacteur $R_1$ comme précédemment décrit.

Le filtre $F_{1b}$ permet l'obtention d'un filtrat clair 9 (100 ml) que l'on introduit dans l'évaporateur $E_1$. L'évaporateur $E_1$ permet de récupérer deux fractions légères. L'une 10 constituée de CHA (100 ml) et l'autre 2b constituée de BCH (2,2 g soit 13,6 mmoles). Le CHA 10 donne les deux alimentations 7 et 3 précédemment décrites. Le BCH 2b alimente le réacteur $R_1$ comme précédemment décrit. Le produit brut 11 (12,09 g) obtenu après traitement dans l'évaporateur $E_1$ sort de $E_1$. Ce produit brut, à la température ambiante, se présente sous forme de deux phases bien distinctes : une phase solide constituée de cristaux de 2,6 DCN et une phase liquide correspondant au mélange de NP, de MCN, de DCN autres que le 2,6 DCN, et de divers autres produits naphtaléniques.

Le produit 11 arrive dans le filtre $F_2$ qui permet de séparer le produit solide 12 (2,1 g) contenant 96 % en poids de 2,6 DCN (6,9 mmoles) et 4 % en poids de MCN (0,4 mmole).

Le filtrat 13 (9,9 g) provenant du filtre $F_2$ est un mélange de composés naphtaléniques, ce mélange étant appauvri en 2,6 DCN (0,7 g soit 2,4 mmoles). Le filtrat 13 est alors soumis au traitement de régénération du naphtalène décrit ci-après.

2 - Régénération du napthalène

Cette phase de régénération utilise le réacteur $R_3$. La constitution de ce réacteur $R_3$ ainsi que les moyens permettant de le chauffer sont analogues à ceux précédemment décrits pour le réacteur $R_1$.

On introduit dans le réacteur $R_3$ les produits suivants :

- . Le filtrat 13 (9,9 g), qui est un mélange de NP, de MCN, de DCN ;
- . Du CHA 14 (50 $cm^3$) ;
- . De la ZE 15 (1,0 g) qui est de la ZE récupérée.

Cette introduction est effectuée dans les conditions ambiantes, la température et la pression dans le réacteur étant respectivement d'environ 20 ° C et 1 bar.

On agite le réacteur à 680 tours/minute, après l'avoir fermé, et on le chauffe de telle sorte que sa température monte de 20 ° C environ à 300 ° C en 18 minutes. On obtient ainsi une suspension du catalyseur dans le milieu réactionnel.

Dès que la température atteint 300 ° C, on arrête à la fois l'agitation et le chauffage, la pression autogène dans le réacteur $R_3$ étant de 50 bars à 300 ° C. Les conditions maximales de température et de pression dans le réacteur, lors de la déalkylation, sont donc respectivement de 300 ° C et 50 bars.

Dans ce réacteur, lors du chauffage, il se produit la réaction suivante, en phase hétérogène liquide/solide :

MCN + DCN → NP + CHE + $H_2$

La déalkylation donnant donc du naphtalène, du cyclohexène et de l'hydrogène.

Lorsque la température du réacteur $R_3$ atteint 20 ° C, à la fin du refroidissement, on ouvre ce réacteur et il se dégage de l'hydrogène 16.

Le mélange hétérogène 17 sortant du réacteur $R_3$ est introduit dans le filtre $F_3$ à deux étages comportant les filtres élémentaires $F_{3a}$ et $F_{3b}$, l'agencement du filtre $F_3$ étant identique à celui du filtre $F_1$.

Le filtre supérieur $F_{3a}$ permet de séparer ZE que l'on lave avec un mélange récupéré 18 (50 $cm^3$) de CHA et de CHE.

La ZE 19 sortant du filtre $F_3$ est introduite dans le four $R_4$ où elle subit le même traitement de régénération que celui réalisé dans le four $R_2$, après alkylation. La zéolithe activée 15 sortant du four $R_4$ est introduite dans le réacteur $R_3$ comme précédemment décrit.

Le liquide 20 (100 $cm^3$) sortant de l'étage inférieur $F_{3b}$ du filtre $F_3$ est un liquide clair constitué par le filtrat du mélange 17 et par le liquide de lavage 18. Ce liquide 20 est introduit dans l'évaporateur $E_2$. L'évaporation donne un mélange de CHA et CHE (100 $cm^3$) dont une partie 18 sert au lavage dans le filtre $F_3$, comme précédemment décrit, et dont l'autre partie 21 (50 $cm^3$) est stockée en vue d'autres utilisations.

Le produit 22 de déalkylation sortant comme résidu de l'évaporateur $E_2$ (6,6 g) est un mélange enrichi en NP (3,1 g, soit 24,2 mmoles). Ce produit se présente à la température ambiante sous forme de deux phases distinctes : une phase solide, constituée de cristaux de NP, et une phase liquide correspondant à un mélange de divers composés naphtaléniques.

Ce produit 22 est recyclé dans le réacteur d'alkylation $R_1$, la phase solide de 22 correspondant ainsi à l'alimentation $1_b$ (NP) et la phase liquide de 22 correspondant ainsi à l'alimentation $1_c$ précédemment décrites dans l'étape d'alkylation.

Le rendement en 2,6 DCN du procédé conforme à l'invention correspond au rapport molaire :

$$\frac{\text{2,6 DCN produit (contenu dans 12)}}{\text{NP d'appoint } 1_a}$$

Ce rapport est de 26,7 %.

Le procédé conforme à l'invention est simple à mettre en oeuvre et il donne un rendement élevé en dicyclohexyl-2,6 naphtalène.

A titre de comparaison, les procédés connus utilisant du chlorure d'aluminium comme catalyseur conduisent aux inconvénients suivants :

- le chlorure d'aluminium forme des complexes avec les dérivés naphtaléniques et tend à souiller les produits, ce qui conduit à la nécessité d'effectuer plusieurs lavages successifs.
- Le chlorure d'aluminium ne peut pas être réutilisé, et il se pose le problème de l'utilisation des sous-produits indésirables.
- Le chlorure d'aluminium ne permet pas de travailler avec un excès de bromocyclohexane ; aussi, en fin de réaction, on a une quantité importante de naphtalène qu'il faut récupérer par distillation avant de traiter le mélange de mono et de polyalkylnaphtalènes, de telle sorte que le rendement en dicyclohexyl-2,6 naphtalène est très faible, inférieur à 5 %.

Le produit 12 obtenu après filtration en $F_2$ est suffisamment riche en 2,6 DCN pour être utilisé tel quel, par exemple pour une synthèse chimique ultérieure. On peut aussi recristalliser le 2,6 DCN contenu dans le produit 12 comme représenté par exemple dans le schéma de la figure 2. Le produit 12 est alors introduit dans le cristallisateur $C_1$ qui reçoit une alimentation 23 (100 ml) d'éthanol bouillant récupéré. On obtient ainsi le 2,6 DCN, 24, pur à 100 % (2,0 g soit 6,7 mmoles). Le filtrat 25 provenant du cristallisateur $C_1$ est introduit dans l'évaporateur $E_3$ qui permet de récupérer l'éthanol 23 que l'on recycle dans le cristallisateur $C_1$. Le résidu 26 (0,1 g) provenant de l'évaporateur $E_3$ est un mélange de MCN et de 2,6 DCN. Ce produit 26 peut être joint au filtrat 13, provenant du filtre $F_2$, pour être introduit dans le réacteur $R_3$ de déalkylation.

Le produit brut 22 sortant de l'évaporateur $E_2$ peut être recyclé directement dans le réacteur d'alkylation $R_1$ comme précédemment décrit. On peut cependant aussi enrichir en NP le produit 22, comme représenté sur le schéma de la figure 3.

Le produit 22 est introduit dans le filtre simple $F_4$. On sépare ainsi 22 en une phase solide 27 (0,9 g) fortement enrichie en NP (89 % soit 6,3 mmoles de NP) et une phase liquide 28 (5,7 g) constituée par un mélange de NP et de divers alkylnaphtalènes, cette phase comportant 40 % de NP. Cette phase 28 est recyclée dans le réacteur $R_3$ de déalkylation. La phase solide 27 est introduite dans le cristallisateur $C_2$ où l'on cristallise le NP par introduction d'éthanol récupéré 29 (25 ml). Le NP 30 recristallisé est recyclé dans le réacteur $R_1$ d'alkylation. Le liquide 31 provenant du cristallisateur $C_2$ est introduit dans l'évaporateur $E_4$ qui permet de séparer 31 en éthanol 29, que l'on recycle dans le cristallisateur $C_2$, et en un résidu 32, constitué de divers alkylnaphtalènes. Le résidu 32 est recyclé avec 13 dans le réacteur $R_3$ de déalkylation.

De préférence, dans le réacteur d'alkylation $R_1$ on a les caractéristiques suivantes, pour la préparation du 2,6 DCN :

- la température maximale est au moins égale à 140 °C et au plus égale à 220 °C ;
- la pression maximale est au moins égale à 5 bars et au plus égale à 30 bars ;

- le rapport molaire

$$\frac{\text{agent d'alkylation}}{\text{naphtalène}}$$

est au moins égal à 1,5 et au plus égal à 4, au début de l'alkylation.

De préférence, dans le réacteur de déalkylation $R_3$, la température maximale est au moins égale à 260 °C et au plus égale à 350 °C, et la pression maximale est au moins égale à 10 bars et au plus égale à 60 bars, pour la préparation du 2,6 DCN.

Bien entendu, l'invention n'est pas limitée aux exemples précédemment décrits, c'est ainsi par exemple que l'invention s'applique à l'alkylation d'autres composés que le naphtalène et/ou à l'utilisation d'autres agents d'alkylation que le bromocyclohexane, dans le cas où le produit alkylé désiré cristallise à partir des produits de la réaction d'alkylation.

D'autres zéolithes que ZF-520 peuvent être utilisées pour l'alkylation et/ou la déalkylation. Ces autres zéolithes, qui ont de préférence la même définition que celle des zéolithes de la demande PCT/CH 90/00178 précitée, peuvent être par exemple les zéolithes ZF-510 et ZF-515 de la société Zéocat, le rapport molaire $S_i0_2/Al_20_3$ étant égal à 20 pour ZF-510 et égal à 30 pour ZF-515.

L'invention s'applique aussi aux cas où l'on alkyle dans La même opération plusieurs composés aromatiques avec plusieurs agents alkylants, et aux cas où l'on utilise plusieurs zéolithes pour l'opération d'alkylation et/ou pour l'opération de déalkylation. En outre, les zéolithes d'alkylation peuvent être les mêmes que les zéolithes de déalkylation, ou différentes de celles-ci.

L'invention s'applique également au cas où l'alkylation et/ou la déalkylation sont réalisées en continu.

## Revendications

**1.** Procédé pour préparer un produit aromatique alkylé (12) par alkylation d'au moins un composé aromatique (1) en utilisant au moins une zéolithe (4) comme catalyseur, cette alkylation donnant plusieurs produits aromatiques alkylés, le procédé étant caractérisé par les points suivants :

a) on fait réagir le composé aromatique (1) de départ avec au moins un agent alkylant (2) en présence d'au moins une zéolithe (4) comme catalyseur dite "zéolithe d'alkylation" ;

b) on sépare ensuite par cristallisation le produit aromatique alkylé (12) désiré à partir des produits de l'alkylation ;

c) on soumet la partie non cristallisée (13) des produits aromatiques alkylés à une déalkylation en présence d'au moins une zéolithe (15) comme catalyseur, dite "zéolithe de déalkylation", de façon à régénérer le composé aromatique ($1_b$) de départ ;

d) on recycle le composé aromatique régénéré ($1_b$) pour le faire réagir à nouveau avec l'agent alkylant (2), en présence de la zéolithe d'alkylation (4).

**2.** Procédé selon la revendication 1, caractérisé en ce que la zéolithe d'alkylation et/ou la zéolithe de déalkylation sont des zéolithes de structure faujasite ayant des ouvertures de pores supérieures à 6,7 Å, le rapport en poids silice/alumine de ces zéolithes étant supérieur à 2,5 et leur taux résiduel en ion-(s) alcalin(s) étant inférieur à 3 % en poids.

**3.** Procédé selon l'une quelconque des revendications 1 ou 2, caractérisé en ce que la zéolithe d'alkylation (4) et la zéolithe de déalkylation (15) sont de même nature, l'alkylation et la déalkylation étant effectuées à des températures différentes.

**4.** Procédé selon l'une quelconque des revendications 1 ou 2, caractérisé en ce que la zéolithe d'alkylation et la zéolithe de déalkylation sont de natures différentes.

**5.** Procédé selon l'une quelconque des revendications 1 à 4, caractérisé en ce que l'alkylation est réalisée en utilisant un solvant (3) du composé (1), de l'agent alkylant (2) et des produits aromatiques alkylés et en ce que la cristallisation est obtenue en évaporant au moins une partie du solvant.

**6.** Procédé selon la revendication 5, caractérisé en ce que le solvant est réutilisé au moins en partie pour l'alkylation après son évaporation.

7. Procédé selon l'une quelconque des revendications 1 à 6, caractérisé en ce que l'on sépare la zéolithe, après alkylation, avant la cristallisation, et en ce que l'on réutilise cette zéolithe pour l'alkylation, après l'avoir activée.

8. Procédé selon l'une quelconque des revendications 1 à 7, caractérisé en ce que l'alkylation est une cycloalkylation.

9. Procédé selon la revendication 8, caractérisé en ce qu'il consiste à alkyler du naphtalène pour obtenir du dicyclohexyl-2,6 naphtalène ; on provoque après alkylation la cristallisation du dicyclohexyl-2,6 naphtalène, que l'on sépare, et la phase liquide séparée par cette cristallisation est soumise à la déalkylation pour obtenir du naphtalène que l'on réutilise pour l'alkylation.

10. Procédé selon la revendication 9, caractérisé par les points suivants : l'alkylation est réalisée en utilisant un solvant du naphtalène, après alkylation on sépare la zéolithe du milieu réactionnel, on réactive cette zéolithe pour la réutiliser dans l'alkylation, on évapore au moins une partie du solvant contenu dans la phase liquide, obtenue par la séparation de la zéolithe, de façon à provoquer la cristallisation du dicyclohexyl-2,6 naphtalène que l'on sépare, et le liquide restant est soumis à l'opération de déalkylation.

11. Procédé selon l'une quelconque des revendications 9 ou 10, caractérisé en ce que, lors de l'alkylation, la température maximale est au moins égale à 140°C et au plus égale à 220°C, et la pression maximale est au moins égale à 5 bars et au plus égale à 30 bars.

12. Procédé selon l'une quelconque des revendications 9 à 11, caractérisé en ce que, au début de l'alkylation, le rapport molaire

$$\frac{\text{agent d'alkylation}}{\text{naphtalène}}$$

est au moins égal à 1,5 et au plus égal à 4.

13. Procédé selon l'une quelconque des revendications 9 à 12, caractérisé en ce que, lors de la déalkylation, la température maximale est au moins égale à 260°C et au plus égale à 350°C, et la pression maximale est au moins égale à 10 bars et au plus égale à 60 bars.

14. Procédé selon l'une quelconque des revendications 1 à 13 caractérisé en ce que la zéolithe d'alkylation et/ou la zéolithe de déalkylation sont en suspension dans le milieu réactionnel.

15. Procédé selon l'une quelconque des revendications 1 à 14, caractérisé en ce qu'il est discontinu.

16. Procédé selon l'une quelconque des revendications 1 à 14, caractérisé en ce qu'il est continu.

## Claims

1. A method for preparing an alkylated aromatic product (12) by alkylation of at least one aromatic compound (1) using at least one zeolite (4) as catalyst, this alkylation giving a plurality of alkylated aromatic products, the method being characterised by the following points:
   (a) the initial aromatic compound (1) is reacted with at least one alkylating agent (2) in the presence of at least one zeolite (4) as catalyst, referred to as "alkylation zeolite";
   (b) the desired alkylated aromatic product (12) is then separated by crystallisation from the products of the alkylation;
   (c) the uncrystallised part (13) of the alkylated aromatic products is subjected to dealkylation in the presence of at least one zeolite (15) as catalyst, referred to as "dealkylation zeolite", so as to regenerate the initial aromatic compound ($1_b$);
   (d) the regenerated aromatic compound ($1_b$) is recycled in order to react it again with the alkylating agent (2), in the presence of the alkylation zeolite (4).

2. A method according to Claim 1, characterised in that the alkylation zeolite and/or the dealkylation zeolite are zeolites of faujasite structure having pore openings of more than 6.7 Å, the silica/alumina weight ratio of these zeolites being greater than 2.5, and their residual concentration in alkaline ion(s) being less than 3% by weight.

3. A method according to any one of Claims 1 or 2, characterized in that the alkylation zeolite (4) and the dealkylation zeolite (15) are of the same nature, the alkylation and the dealkylation being effected at different temperatures.

4. A method according to any one of Claims 1 or 2, characterized in that the alkylation zeolite and the dealkylation zeolite are of different natures.

5. A method according to any one of Claims 1 to 4, characterized in that the alkylation is effected with the use of a solvent (3) of the compound (1), the alkylating agent (2) and the alkylated aromatic products, and in that the crystallization is obtained by evaporating at least a part of the solvent.

6. A method according to Claim 5, characterized in that the solvent is re-used at least in part for the alkylation after its evaporation.

7. A method according to any one of Claims 1 to 6, characterized in that the zeolite is removed, after alkylation, before the crystallization, and in that said zeolite is re-used for the alkylation, after it has been activated.

8. A method according to any one of Claims 1 to 7, characterized in that the alkylation is a cycloalkylation.

9. A method according to Claim 8, characterized in that it consists in alkylating naphthalene in order to obtain 2,6-dicyclohexyl naphthalene; after alkylation, the crystallization of the 2,6-dicyclohexyl naphthalene is effected, which is separated, and the liquid phase separated by this crystallization is subjected to dealkylation in order to obtain naphthalene, which is re-used for the alkylation.

10. A method according to Claim 9, characterized by the following points: the alkylation is effected with the use of a solvent for the naphthalene, the zeolite is separated from the reaction medium after alkylation, this zeolite is reactivated in order to re-use it in the alkylation, at least a part of the solvent contained in the liquid phase obtained by the separation of the zeolite is evaporated so as to cause the crystallization of the 2,6-dicyclohexyl naphthalene which is separated, and the remaining liquid is subjected to the dealkylation operation.

11. A method according to any one of Claims 9 or 10, characterized in that upon the alkylation the maximum temperature is at least equal to 140 °C and at most equal to 220 °C, and the maximum pressure is at least equal to 5 bar and at most equal to 30 bar.

12. A method according to any one of Claims 9 to 11, characterized in that at the start of the alkylation the molar ratio of alkylating agent/naphthalene is at least equal to 1.5 and at most equal to 4.

13. A method according to any one of Claims 9 to 12, characterized in that upon the dealkylation, the maximum temperature is at least equal to 260 °C and at most equal to 350 °C, and the maximum pressure is at least equal to 10 bar and at most equal to 60 bar.

14. A method according to any one of Claims 1 to 13, characterized in that the alkylation zeolite and/or the dealkylation zeolite are suspended in the reaction medium.

15. A method according to any one of Claims 1 to 14, characterized in that it is discontinuous.

16. A method according to any one of Claims 1 to 14, characterized in that it is continuous.

**Patentansprüche**

1. Verfahren zur Herstellung eines alkylierten aromatischen Produktz (12) durch Alkylierung mindestens einer aromatischen Verbindung (1) unter Verwendung mindestens eines Zeoliths (4) als Katalysator, wobei diese Alkylierung mehrere alkylierte aromatizche Verbindungen ergibt,
gekennzeichnet durch folgende Merkmale:
    a) Umsetzung der als Ausgangsmaterial dienenden aromatizchen Verbindung (1) mit mindestens einem Alkylierungsmittel (2) in Gegenwart mindestens eines als "Alkylierungszeolith" bezeichneten Zeoliths (4) als Katalysator;
    b) Abtrennung des angestrebten alkylierten aromatischen Produkts (12) aus den Alkylierungsprodukten durch Kristallisation;
    c) Dealkylierung des nichtkristallisierten Teils (13) der alkylierten aromatischen Produkte in Gegenwart mindestens eines als "Dealkylierungszeolith" bezeichneten Zeoliths (15) als Katalysator, um so die als Ausgangsmaterial eingesetzte aromatische Verbindung (1b) zu regenerieren;
    d) Rückführung der regenerierten aromatischen Verbindung (1b) zur neuerlichen Umsetzung mit dem Alkylierungsmittel (2) in Gegenwart des Alkylierungszeoliths (4).

2. Verfahren nach Anspruch 1, dadurch gekennzeichnet, daß der Alkylierungszeolith und/oder der Dealkylierungszeolith Zeolithe mit Faujasit-Struktur mit einer Porenweite von mehr als 6,7 Å sind, wobei das Gewichtsverhältnis von Siliciumdioxid zu Aluminiumoxid dieser Zeolithe mehr als 2,5 beträgt und der Restgehalt an einem oder mehreren Alkaliionen weniger als 3 Gew.-% beträgt.

3. Verfahren nach Anspruch 1 oder 2, dadurch gekennzeichnet, daß der Alkylierungszeolith (4) und der Dealkylierungszeolith (15) von gleicher Art sind, wobei die Alkylierung und die Dealkylierung bei unterschiedlichen Temperaturen vorgenommen werden.

4. Verfahren nach Anspruch 1 oder 2, dadurch gekennzeichnet, daß der Alkylierungszeolith und der Dealkylierungszeolith unterschiedliche Zeolithe sind.

5. Verfahren nach einem der Ansprüche 1 bis 4, dadurch gekennzeichnet, daß die Alkylierung unter Verwendung eines Lösungsmittels (3) der aromatischen Verbindung (1), des Alkylierungsmittels (2) und der alkylierten aromatischen Produkte durchgeführt wird und die Kristallisation durch Abdampfen mindestens eines Teils des Lösungsmittels vorgenommen wird.

6. Verfahren nach Anspruch 5, dadurch gekennzeichnet, daß das Lösungsmittel nach dem Abdampfen mindestens zum Teil zur Alkylierung wiederverwendet wird.

7. Verfahren nach einem der Ansprüche 1 bis 6, dadurch gekennzeichnet, daß der Zeolith nach der Alkylierung und vor der Kristallisation abgetrennt wird und dieser Zeolith nach Aktivierung zur Alkylierung wiederverwendet wird.

8. Verfahren nach einem der Ansprüche 1 bis 7, dadurch gekennzeichnet, daß die Alkylierung eine Cycloalkylierung ist.

9. Verfahren nach Anspruch 8, dadurch gekennzeichnet, daß
zur Herstellung von 2,6-Dicyclohexylnaphthalin Naphthalin alkyliert wird,
die Kristallisation des 2,6-Dicyclohexylnaphthalins nach der Alkylierung vorgenommen wird, das abgetrennt wird,
und die durch diese Kristallisation abgetrennte flüssige Phase der Dealkylierung unterzogen wird, um Naphthalin zu gewinnen, das zur Alkylierung wiederverwendet wird.

10. Verfahren nach Anspruch 9, gekennzeichnet durch folgende Merkmale:
Die Alkylierung wird unter Verwendung eines Lösungsmittels für Naphthalin durchgeführt;
nach der Alkylierung wird der Zeolith vom Reaktionsmedium abgetrennt und zur Wiederverwendung bei der Alkylierung reaktiviert;
mindestens ein Teil des in der durch Abtrennung des Zeoliths erhaltenen flüssigen Phase enthaltenen Lösungsmittels wird abgedampft, um die Kristallisation des 2,6-Dicyclohexylnaphthalins hervorzurufen, das abgetrennt wird;

die verbleibende Flüssigkeit wird der Dealkylierung unterzogen.

**11.** Verfahren nach Anspruch 9 oder 10, dadurch gekennzeichnet, daS bei der Alkylierung die maximale Temperatur mindestens gleich 140 °C und höchstens gleich 220 °C und der maximale Druck mindestens gleich 5 bar und höchstens gleich 30 bar sind.

**12.** Verfahren nach einem der Ansprüche 9 bis 11, dadurch gekennzeichnet, daß das Molverhältnis

$$\frac{\text{Alkylierungsmittel}}{\text{Naphthalin}}$$

zu Beginn der Alkylierung mindestens gleich 1,5 und höchstens gleich 4 ist.

**13.** Verfahren nach einem der Ansprüche 9 bis 12, dadurch gekennzeichnet, daß bei der Dealkylierung die maximale Temperatur mindestens gleich 260 °C und höchstens gleich 350 °C und der maximale Druck mindestens gleich 10 bar und höchstens gleich 60 bar sind.

**14.** Verfahren nach einem der Ansprüche 1 bis 13, dadurch gekennzeichnet, daß der Alkylierungszeolith und/oder der Dealkylierungszeolith in Suspension im Reaktionsmedium vorliegen.

**15.** Verfahren nach einem der Ansprüche 1 bis 14, dadurch gekennzeichnet, daß es diskontinuierlich durchgeführt wird.

**16.** Verfahren nach einem der Ansprüche 1 bis 14, dadurch gekennzeichnet, daß es kontinuierlich durchgeführt wird.

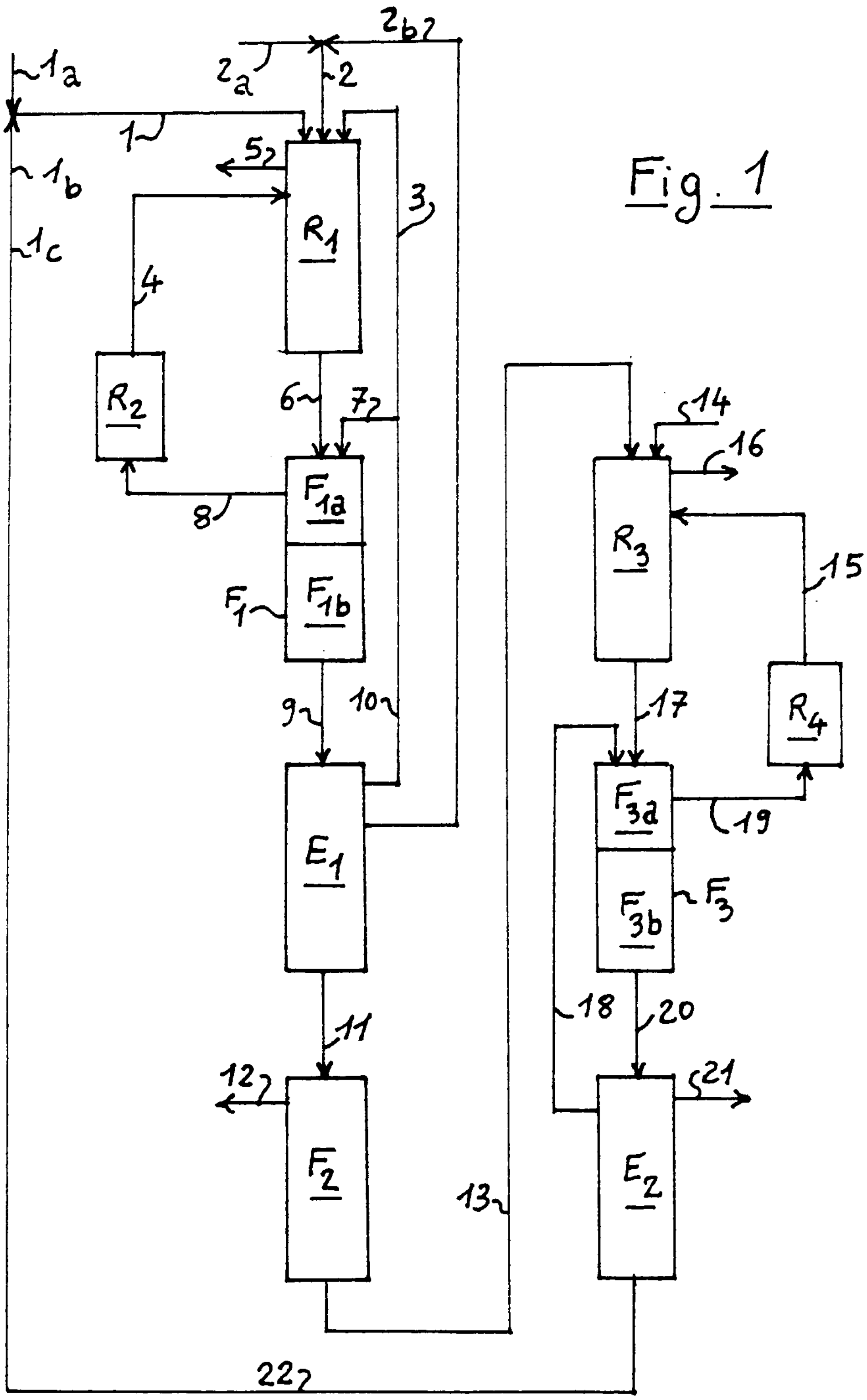
Fig. 1
1a
1b
1c
1
2a
2
2b
3
4
5
R1
R2
6
7
8
F1a
F1b
F1
9
10
E1
11
12
F2
13
14
16
R3
15
17
R4
F3a
19
F3b
F3
18
20
21
E2
22

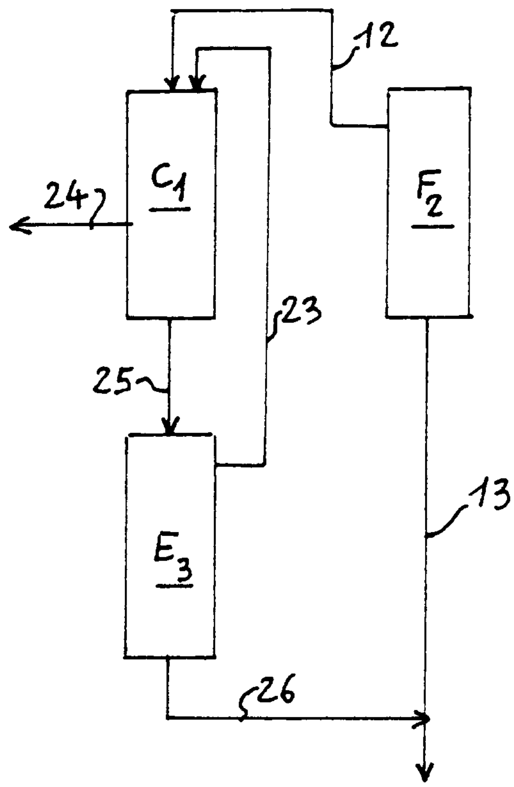


Fig. 2

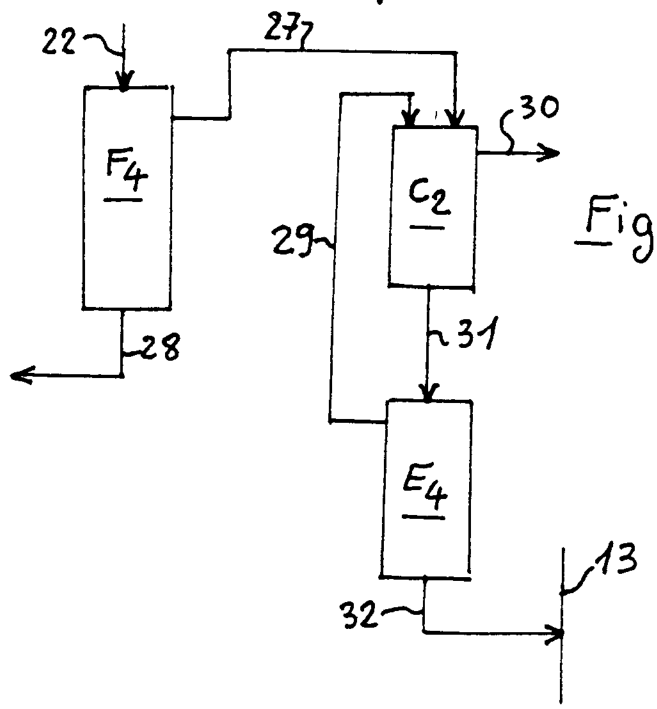


Fig. 3